(19) Europäisches Patentamt
European Patent Office
Office européen des brevets

(11) Publication number: **0 129 846 B1**

# (12) EUROPEAN PATENT SPECIFICATION

(45) Date of publication of patent specification: **08.05.91**

(51) Int. Cl.5: **C07D 231/38**, C07D 401/06, C07D 405/06, C07D 409/06

(21) Application number: **84107102.0**

(22) Date of filing: **20.06.84**

## (54) (3-Amino-1H-pyrazol-4-yl)(aryl)methanones.

(30) Priority: **23.06.83 US 507317**

(43) Date of publication of application:
**02.01.85 Bulletin 85/01**

(45) Publication of the grant of the patent:
**08.05.91 Bulletin 91/19**

(84) Designated Contracting States:
**AT BE CH DE FR GB IT LI NL SE**

(56) References cited:
**DE-A- 2 557 514**
**US-A- 4 178 449**

(73) Proprietor: **AMERICAN CYANAMID COMPANY**
**1937 West Main Street P.O. Box 60**
**Stamford Connecticut 06904-0060(US)**

(72) Inventor: **Tomcufcik, Andrew Stephen**
**48 Dearborn Drive**
**Old Tappan, NJ 07675(US)**
Inventor: **Dusza, John Paul**
**24 Convent Road**
**Nanuet, NY 10954(US)**

(74) Representative: **Wächtershäuser, Günter, Dr.**
**Tal 29**
**W-8000 München 2(DE)**

EP 0 129 846 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to new organic compounds which may be represented by the following structural formula:

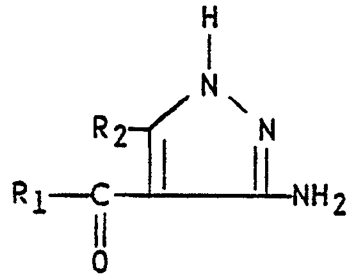

wherein $R_1$ is selected from the group consisting of phenyl substituted by one or two of the group selected from halogen, alkyl($C_1$-$C_3$) and alkoxy($C_1$-$C_3$); phenyl substituted by one of the group consisting of dialkylamino($C_1$-$C_3$), methylenedioxy, alkylthio($C_1$-$C_3$), alkylsulfonyl($C_1$-$C_3$), substituted arylsulfonyl, amino, alkanoyl($C_1$-$C_3$)amino, substituted aroylamino, trifluoromethyl and phenyl; pyridinyl; pyridinyl substituted by one or two of the group selected from halogen, alkyl($C_1$-$C_3$) and alkoxy($C_1$-$C_3$); thienyl; thienyl substituted by one or two of the group selected from halogen, alkyl($C_1$-$C_3$) and alkoxy($C_1$-$C_3$);furanyl; naphthalenyl; and pyrazinyl; and $R_2$ is selected from the group consisting of hydrogen and alkyl($C_1$-$C_3$).

The compounds of the present invention may be readily prepared as set forth in the following reaction scheme:

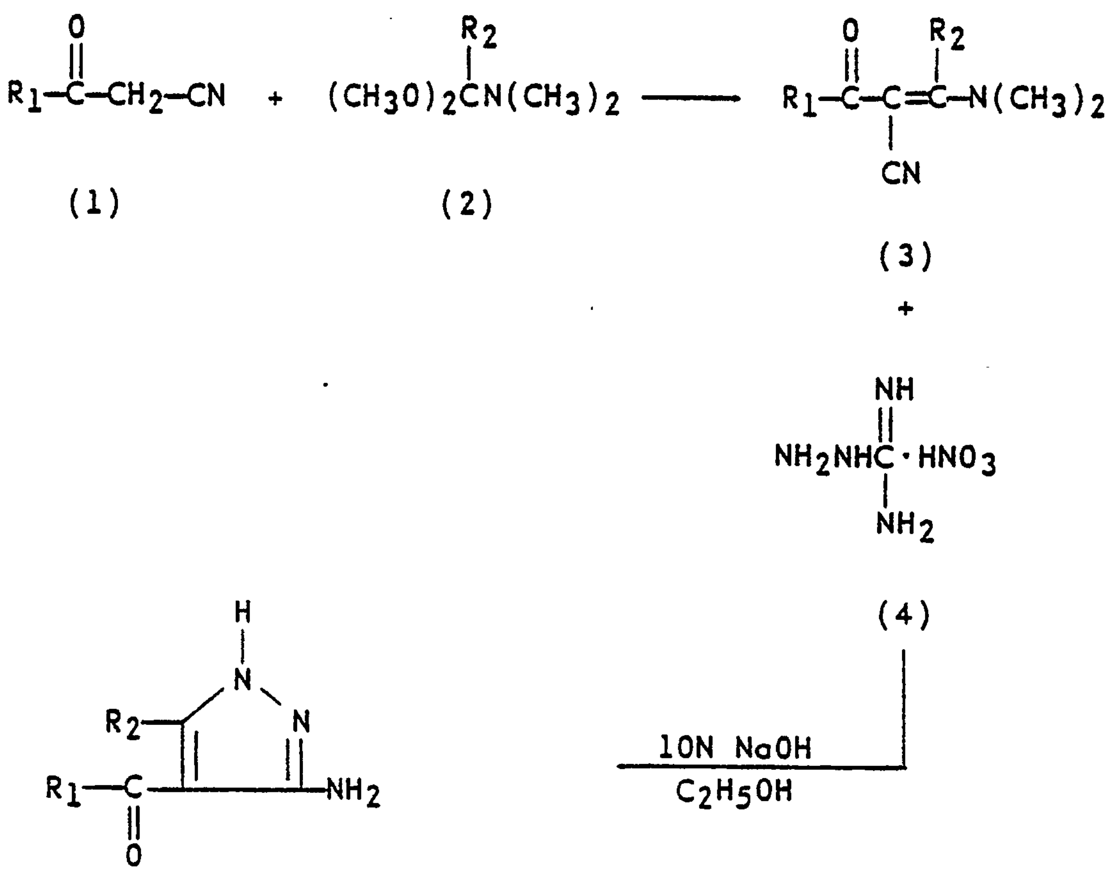


(1) (2) (3) (4) (5)

In accordance with the above reaction scheme an appropriately substituted acetonitrile (1), where $R_1$ is as described above is reacted with a dimethylamide dimethylacetal (2) where $R_2$ is as described above.

The resulting exothermic reaction produces a crystalline solid which is recovered by evaporation and dissolved in methylene chloride. This solution is passed through hydrous magnesium silicate and hexane is added to the refluxing eluate, giving the [(α-dimethylamino)methylene]-β-oxoarylpropanenitrile (3) which is then reacted with aminoguanidine nitrate (4) in the presence of 10N sodium hydroxide and a to dryness and crystallized from water, ethanol or other suitable solvent, giving (5). The aminoguanidine nitrate may be replaced by other salts of aminoguanidine, such as the hydrochloride, sulfate, and the like. Alternatively, the aminoguanidine salt-sodium hydroxide combination may be replaced by an equivalent of aminoguanidine bicarbonate or thiosemicarbazide, both reagents resulting in the formation of (5).

The (3-amino-1H-pyrazol-4-yl)(aryl)methanones find utility as intermediates in the preparation of therapeutic aryl and heteroaryl[7-(aryl and heteroaryl)pyrazolo[1,5-a]pyrimidin-3-yl]methanones which are disclosed in the United States patent 4,178,449. The EP-A2 129 847 also describes similar compounds. Such final products are useful as anxiolytic or antiepileptic agents as well as sedative-hypnotic and skeletal muscle relaxant agents. The novel compounds of the instant invention additionally have utility as anxiolytic agents.

The following non-limiting examples illustrate the preparation of the compounds of the present invention.

Example 1

α-[(Dimethylamino)methylene]-β-oxo-2-furanepropanenitrile

A 50 ml portion of dimethylformamide dimethylacetal was added to 25 g of solid β-oxo-2-furanepropanenitrile. This exothermic reaction produced yellow crystals. After one hour the volatiles were removed under reduced pressure and the residue was dissolved in methylene chloride. This solution was passed through a short pad of hydrous magnesium silicate. The eluate was refluxed with the gradual addition of hexane to the point of turbidity. Cooling and filtration gave 35.2 g of the desired compound, mp 117-125 °C.

Example 2

α-[(Dimethylamino)methylene]-β-oxo-benzenepropanenitrile

A 100 g portion of β-oxo-benzenepropanenitrile was placed in a 500 ml round-bottom flask and 110 ml of dimethylformamide dimethylacetal was added in one portion. The reaction mixture became warm and a homogeneous dark yellow solution resulted, which then solidified. After cooling to room temperature, hexane was added giving crystals which were recovered by filtration. This material (143.6 g, mp 102-105 °C) is suitable for the subsequent reaction without further purification.

An analytical sample of this compound was obtained by dissolution in methylene chloride followed by passage through a short column of hydrous magnesium silicate, concentration of the eluate with the gradual addition of hexane until crystallization occurred, cooling and collection by filtration, mp 111-113 °C.

Following the general procedures of Examples 1 or 2, the following compounds of Examples 3-31 , shown in Table I were prepared.

**TABLE I**

| Example | Acetonitrile | Compound | MP°C |
|---|---|---|---|
| 3 | β-oxo-4-fluorobenzenepropane-nitrile | α-[(dimethylamino)methylene]-β-oxo-4--fluorobenzenepropanenitrile | 142-145 |
| 4 | β-oxo-(3-trifluoromethyl)benzene-propanenitrile | α-[(dimethylamino)methylene]-β-oxo-3-(trifluoromethyl)benzenepropanenitrile | 93-96 |
| 5 | β-oxo-4-pyridinepropanenitrile | α-[(dimethylamino)methylene]-β-oxo-4-pyridinepropanenitrile | 127-128 |
| 6 | β-oxo-2-thiophenepropanenitrile | α-[(dimethylamino)methylene]-β-oxo-2-thiophenepropanenitrile | 136-140 |
| 7 | β-oxo-4-methylbenzenepropane-nitrile | α-[(dimethylamino)methylene]-β-oxo-4--methylbenzenepropanenitrile | 132-135 |
| 8 | β-oxo-2-pyridinepropanenitrile | α-[(dimethylamino)methylene]-β-oxo-2-pyridinepropanenitrile | 88-90 |
| 9 | β-oxo-3-fluorophenylpropanenitrile | α-[(dimethylamino)methylene]-β-oxo-3-fluorophenylpropanenitrile | 63-68 |
| 10 | β-oxo-2-chlorophenylpropanenitrile | α-[(dimethylamino)methylene]-β-oxo-2-chlorophenylpropanenitrile | 152-154 |

TABLE I (continued)

| Example | Acetonitrile | Compound | MP°C |
|---|---|---|---|
| 11 | β-oxo-3-furanylpropanenitrile | α-[(dimethylamino)methylene]-β-oxo-3-furanylpropanenitrile | 98-103 |
| 12 | β-oxo-3,4,5-trimethoxyphenylpropanenitrile | α-[(dimethylamino)methylene]-β-oxo-3,4,5-trimethoxyphenylpropanenitrile | 138-140 |
| 13 | β-oxo-3,4-dimethoxyphenylpropanenitrile | α-[(dimethylamino)methylene]-β-oxo-3,4-dimethoxyphenylpropanenitrile | glassy solid |
| 14 | β-oxo-3-methylphenylpropanenitrile | α-[(dimethylamino)methylene]-β-oxo-3-methylphenylpropanenitrile | 74-80 |
| 15 | β-oxo-3,5-dimethoxyphenylpropanenitrile | α-[(dimethylamino)methylene]-β-oxo-3,5-dimethoxyphenylpropanenitrile | 125-127 |
| 16 | β-oxo-4-chlorophenylpropanenitrile | α-[(dimethylamino)methylene]-β-oxo-4-chlorophenylpropanenitrile | 118-121 |
| 17 | β-oxo-4-methoxyphenylpropanenitrile | α-[(dimethylamino)methylene]-β-oxo-4-methoxyphenylpropanenitrile | 128-130 |
| 18 | β-oxo-2-fluorophenylpropanenitrile | α-[(dimethylamino)methylene]-β-oxo-2-fluorophenylpropanenitrile | 62-74 |

TABLE I (continued)

| Ex. | Acetonitrile | Compound | MP°C |
|---|---|---|---|
| 19 | β-oxo-3-methoxyphenylpropanenitrile | α-[(dimethylamino)methylene]-β-oxo-3-methoxyphenylpropanenitrile | Syrup |
| 20 | β-oxo-[4-(trifluoromethyl)phenyl]propanenitrile | α-[(dimethylamino)methylene]-β-oxo-[4-(trifluoromethyl)phenyl]propanenitrile | 122-123 |
| 21 | β-oxo-3-chlorophenylpropanenitrile | α-[(dimethylamino)methylene]-β-oxo-3-chlorophenylpropanenitrile | Syrup |
| 22 | β-oxo-2,5-dichlorophenylpropanenitrile | α-[(dimethylamino)methylene]-β-oxo-2,5-dichlorophenylpropanenitrile | 140-143 |
| 23 | β-oxo-2-methylphenylpropanenitrile | α-[(dimethylamino)methylene]-β-oxo-2-methylphenylpropanenitrile | 82-84 |
| 24 | β-oxo-[4-(dimethylamino)phenyl]propanenitrile | α-[(dimethylamino)methylene]-β-oxo-[4-(dimethylamino)phenyl]propanenitrile | 208-210 |
| 25 | β-oxo-2-methoxyphenylpropanenitrile | α-[(dimethylamino)methylene]-β-oxo-2-methoxyphenylpropanenitrile | 105-115 |
| 26 | β-oxo-[3,4-(methylenedioxy)phenyl]propanenitrile | α-[(dimethylamino)methylene]-β-oxo-[3,4-(methylenedioxy)phenyl]propanenitrile | 118-124 |
| 27 | β-oxo-4-ethoxyphenylpropanenitrile | α-[(dimethylamino)methylene]-β-oxo-4-ethoxyphenylpropanenitrile | 110-115 |
| 28 | β-oxo-4-ethylphenylpropanenitrile | α-[(dimethylamino)methylene]-β-oxo-4-ethylphenylpropanenitrile | 48-54 |

TABLE I (continued)

| Ex. | Acetonitrile | Compound | MP°C |
|---|---|---|---|
| 29 | β-oxo-2-naphthalenylpropanenitrile | α-[(dimethylamino)methylene]-β-oxo-2-naphthalenylpropanenitrile | 115-118 |
| 30 | β-oxo-5-methyl-2-thienylpropanenitrile | α-[(dimethylamino)methylene]-β-oxo-5-methyl-2-thienylpropanenitrile | 152-153 |
| 31 | β-oxo-2-thienylpropanenitrile | α-[(dimethylamino)methylene]-β-oxo-2-thienylpropanenitrile | 118-120 |

Example 32

α-[(1-Dimethylamino)ethylidene]-β-oxo-phenylpropanenitrile

A solution of 14.5 grams of benzoylacetonitrile in 100 ml of chloroform was cooled to -10° C and stirred as a solution of 13.3 g of N,N-dimethylacetamide dimethylacetal in 20 ml of chloroform was added dropwise during 15 minutes. The reaction temperature was not allowed to exceed -5° C. Stirring was continued at -5° to -10° C for two hours after addition ended. The resultant reaction mixture was dissolved in 150 ml of benzene and the solution passed through a layer of hydrous magnesium silicate. Evaporation of the filtrate in air left a yellow residue which was purified by recrystallization from a mixture of benzene and low boiling petroleum ether; yield, 5.8 g, mp 105°-106° C.

Example 33

(3-Amino-1H-pyrazol-4-yl) (2-furanyl)methanone

A reaction mixture comprising 19.0 g of α-[(dimethylamino)methylene]-β-oxo-2-furanepropanenitrile, 16.1 g of aminoguanidine nitrate, 250 ml of ethanol and 11.0 ml of 10N sodium hydroxide was refluxed for 6 hours and then evaporated to dryness. Water was added to the crude residue and the precipitated solid was collected, giving 17.0 g of the desired product, mp 153-155° C.

Example 34

(3-Amino-1H-pyrazol-4-yl) phenylmethanone

A reaction mixture comprising 73.36 g of α-[(dimethylamino)methylene]-β-oxo-2-benzenepropanenitrile, 63.45 g of aminoguanidine nitrate, 500 ml of ethanol and 36.6 ml of 10N sodium hydroxide was refluxed for 10 hours and then cooled. The resulting precipitate was collected and washed with water, giving 17.1 g of the desired product, mp 177-179 °C.

When the aminoguanidine nitrate-10N sodium hydroxide combination was replaced by an equivalent of aminoguanidine bicarbonate, the identical product was obtained as shown by its melting point, elemental analysis and infrared and nuclear magnetic resonance absorption spectra. A similar result was obtained when an equivalent of thiosemicarbazide replaced the aminoguanidine nitrate-10N sodium hydroxide combination.

Following the general procedures of Examples 33 and 34, employing the compounds of Examples 3-32 and the appropriate guanidine derivatives, the products of Examples 35-64, given in Table II, were prepared.

**TABLE II**

| Example | Starting Material of Example | Product | MP°C |
|---|---|---|---|
| 35 | 3 | (3-amino-1H-pyrazol-4-yl)(4-fluorophenyl)methanone | 172-175 |
| 36 | 4 | (3-amino-1H-pyrazol-4-yl)[3-(trifluoromethyl)phenyl]methanone | 134-136 |
| 37 | 5 | (3-amino-1H-pyrazol-4-yl)(4-pyridinyl)methanone | 275-277 |
| 38 | 6 | (3-amino-1H-pyrazol-4-yl)(2-thienyl)methanone | 144-145 |
| 39 | 32 | (3-amino-5-methyl-1H-pyrazol-4-yl) phenylmethanone | 179-180 |
| 40 | 7 | (3-amino-1H-pyrazol-4-yl)(4-methylphenyl)methanone | 177-179 |
| 41 | 8 | (3-amino-1H-pyrazol-4-yl)(2-pyridinyl)methanone | 118-120 |
| 42 | 9 | (3-amino-1H-pyrazol-4-yl)(3-fluorophenyl)methanone | 188-189 |

**TABLE II (continued)**

| Example | Starting Material of Example | Product | MP°C |
|---|---|---|---|
| 43 | 10 | (3-amino-1H-pyrazol-4-yl)(2-chlorophenyl)methanone | glassy solid |
| 44 | 11 | (3-amino-1H-pyrazol-4-yl)(3-furanyl)methanone | 211-215 |
| 45 | 12 | (3-amino-1H-pyrazol-4-yl)(3,4,5-trimethoxyphenyl)methanone | 199-201 |
| 46 | 13 | (3-amino-1H-pyrazol-4-yl)(3,4-dimethoxyphenyl)methanone | 108-109 |
| 47 | 14 | (3-amino-1H-pyrazol-4-yl)(3-methylphenyl)methanone | 137-139 |
| 48 | 15 | (3-amino-1H-pyrazol-4-yl)(3,5-dimethoxyphenyl)methanone | 91-92 |
| 49 | 16 | (3-amino-1H-pyrazol-4-yl)(4-chlorophenyl)methanone | 235-237 |
| 50 | 17 | (3-amino-1H-pyrazol-4-yl)(4-methoxyphenyl)methanone | 172-174 |
| 51 | 18 | (3-amino-1H-pyrazol-4-yl)(2-fluorophenyl)methanone | glassy solid |

## TABLE II (continued)

| Ex. | Starting Material of Example | Product | MP°C |
|---|---|---|---|
| 52 | 19 | (3-amino-1*H*-pyrazol-4-yl)-(3-methoxyphenyl)methanone | 96-98 |
| 53 | 20 | (3-amino-1*H*-pyrazol-4-yl)-[4-(trifluoromethyl)phenyl)methanone | 172-174 |
| 54 | 21 | (3-amino-1*H*-pyrazol-4-yl)-(3-chlorophenyl)methanone | 229-230 |
| 55 | 22 | (3-amino-1*H*-pyrazol-4-yl)-(2,5-dichlorophenyl)methanone | Syrup |
| 56 | 23 | (3-amino-1*H*-pyrazol-4-yl)-(2-methylphenyl)methanone | Glass |
| 57 | 24 | (3-amino-1*H*-pyrazol-4-yl)-[4-(dimethylamino)phenyl]-methanone | 240-243 |
| 58 | 25 | (3-amino-1*H*-pyrazol-4-yl)-(2-methoxyphenyl)methanone | Glass |
| 59 | 26 | (3-amino-1*H*-pyrazol-4-yl)-[3,4-(methylenedioxy)phenyl]methanone | 228-230 |
| 60 | 27 | (3-amino-1*H*-pyrazol-4-yl)-(4-ethoxyphenyl)methanone | 155-156 |
| 61 | 28 | (3-amino-1*H*-pyrazol-4-yl)-(4-ethylphenyl)methanone | 108-109 |

TABLE II (continued)

| Ex. | Starting Material of Example | Product | MP°C |
|---|---|---|---|
| 62 | 29 | (3-amino-1H-pyrazol-4-yl)-(2-naphthalenyl)methanone | 215-217 |
| 63 | 30 | (3-amino-1H-pyrazol-4-yl)-(5-methyl-2-thienyl)methanone | 165-166 |
| 64 | 31 | (3-amino-1H-pyrazol-4-yl)-(2-thienyl)methanone | 181-183 |

## Claims

1. A compound of the formula:

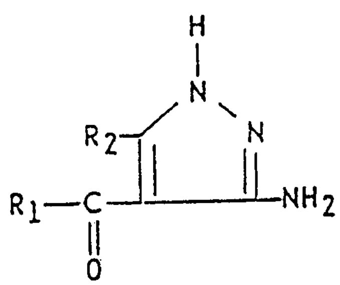

wherein $R_1$ is phenyl substituted by one or two of halogen, alkyl($C_1$-$C_3$) or alkoxy($C_1$-$C_3$); phenyl substituted by one of dialkylamino($C_1$-$C_3$), methylenedioxy, alkylthio($C_1$-$C_3$), alkylsulfonyl($C_1$-$C_3$), substituted arylsulfonyl, amino, alkanoyl($C_1$-$C_3$)amino, substituted aroylamino, trifluoromethyl or phenyl; pyridinyl; pyridinyl substituted by one or two of halogen, alkyl($C_1$-$C_3$)or alkoxy($C_1$-$C_3$); thienyl; thienyl substituted by one or two of halogen, alkyl($C_1$-$C_3$) or alkoxy($C_1$-$C_3$); furanyl; naphthalenyl; or pyrazinyl; and $R_2$ is hydrogen or alkyl($C_1$-$C_3$).

2. The compound according to Claim 1, (3-amino-1H-pyrazol-4-yl)(2-furanyl)methanone; (3-amino-1H-pyrazol-4-yl)(4-chlorophenyl)methanone; (3-amino-1H-pyrazol-4-yl)(4-fluorophenyl)methanone; (3-amino-1H-pyrazol-4-yl)-(4-methoxyphenyl)methanone; (3-amino-1H-pyrazol-4-yl)[3-(trifluoromethyl)phenyl]-methanone; (3-amino-1H-pyrazol-4-yl)(4-pyridinyl)methanone; (3-amino-1H-pyrazol-4-yl)-(2-thienyl)-methanone; (3-amino-5-methyl-1H-pyrazol-4-yl)-phenylmethanone; (3-amino-1H-pyrazol-4-yl)(4-methylphenyl)methanone; (3-amino-1H-pyrazol-4-yl)(2-pyridinyl)-methanone; (3-amino-1H-pyrazol-4-yl)(3-fluorophenyl)methanone; (3-amino-1H-pyrazol-4-yl)(3-furanyl)methanone; (3-amino-1H-pyrazol-4-yl)-(3,4,5-trimethoxyphenyl)methanone; (3-amino-1H-pyrazol-4-yl)(3,4-dimethoxyphenyl)methanone; (3-amino-1H-pyrazol-4-yl)(3-methylphenyl)methanone; (3-amino-1H-pyrazol-4-yl)(3,5-dimethoxyphenyl)-methanone; or (3-amino-1H-pyrazol-4-yl)(2-fluorophenyl)methanone.

**3.** A process of preparing a compound of the formula:

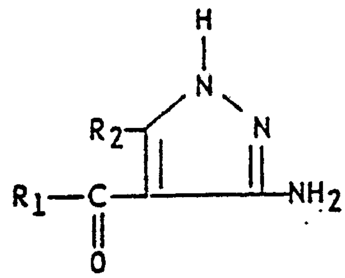

wherein $R_1$ and $R_2$ are as defined in claim 1, which comprises reacting an appropriately substituted acetonitrile of the formula

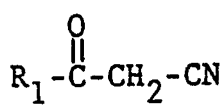

(where $R_1$ is as described above) with an N,N-dimethylamide dimethylacetal producing, after an exothermic reaction, a crystalline solid, recovering said crystalline solid, dissolving in methylene chloride, passing through hydrous magnesium silicate, adding hexane to the refluxing eluate, precipitating an [(α-dimethylamino)methylene]-β-oxoarylpropanenitrile of the formula

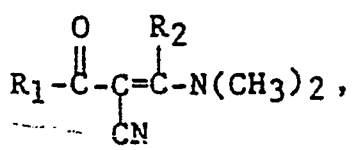

reacting with aminoguanidine salt in a lower alkanol solution of 10N sodium hydroxide at reflux for 6-10 hours, giving the desired product.

Claim for the following Contracting State: AT

**1.** A process of preparing a compound of the formula:

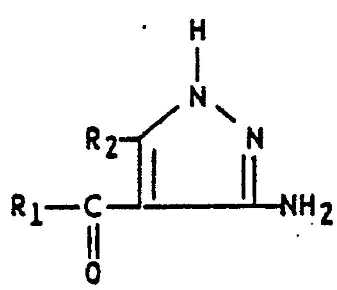

wherein $R_1$ is phenyl substituted by one or two of halogen, alkyl($C_1$-$C_3$) or alkoxy($C_1$-$C_3$); phenyl substituted by one of dialkylamino($C_1$-$C_3$), methylenedioxy, alkylthio($C_1$-$C_3$), alkylsulfonyl($C_1$-$C_3$), substituted arylsulfonyl, amino, alkanoyl($C_1$-$C_3$)amino, substituted aroylamino, trifluoromethyl or phenyl; pyridinyl; pyridinyl substituted by one or two of halogen, alkyl($C_1$-$C_3$)or alkoxy($C_1$-$C_3$); thienyl; thienyl substituted by one or two of halogen, alkyl($C_1$-$C_3$) or alkoxy($C_1$-$C_3$); furanyl; naphthalenyl; or pyrazinyl; and $R_2$ is hydrogen or alkyl($C_1$-$C_3$)which comprises reacting an appropriately substituted acetonitrile of the formula

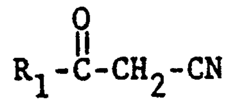

(where $R_1$ is as described above) with an N,N-dimethylamide dimethylacetal producing, after an exothermic reaction, a crystalline solid, recovering said crystalline solid, dissolving in methylene chloride, passing through hydrous magnesium silicate, adding hexane to the refluxing eluate, precipitating an [($\alpha$-dimethylamino)methylene]-$\beta$-oxoarylpropanenitrile of the formula

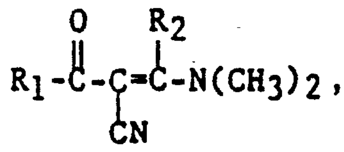

reacting with aminoguanidine salt in a lower alkanol solution of 10N sodium hydroxide at reflux for 6-10 hours, giving the desired product.

## Revendications

1. Un composé de formule :

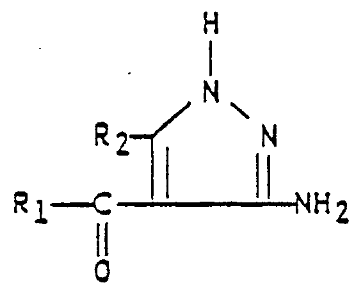

dans laquelle $R_1$ est choisi parmi un groupe phényle substitué par 1 ou 2 substituants choisis parmi les halogènes et les groupes alkyles en $C_1$-$C_3$ et les groupes alcoxy en $C_1$-$C_3$ ; un groupe phényle substitué par un substituant choisi parmi un grope dialkylamino en $C_1$-$C_3$, un groupe méthylènedioxy, un groupe alkylthio en $C_1$-$C_3$, un groupe alkylsulfonyle en $C_1$-$C_3$, un groupe arylsulfonyle substitué, un groupe amino, un groupe alcanoylamino en $C_1$-$C_3$, un groupe aroylamino substitué, un groupe trifluorométhyle et un groupe phényle ; un groupe pyridyle ; un groupe pyridyle substitué par 1 ou 2 substituants choisis parmi les halogènes et les groupes alkyles en $C_1$-$C_3$ et alcoxy en $C_1$-$C_3$ ; le groupe thiényle ; un groupe thiényle substitué par 1 ou 2 substituants choisis parmi les halogènes et les groupes alkyles en $C_1$-$C_3$ et alcoxy en $C_1$-$C_3$ ; un groupe furyle ; un groupe naphtyle ; et un groupe pyrazinyle ; et $R_2$ est choisi parmi l'hydrogène et les groupes alkyles en $C_1$-$C_3$.

2. Le composé selon la revendication 1, qui est la (3-amino-1H-pyrazole-4-yl)-(2-furyl)cétone, la (3-amino-1H-pyrazole-4-yl)-(4-chlorophényl)cétone, la (3-amino-1H-pyrazole-4-yl)-(4-fluorophényl)cétone, la (3-amino-1H-pyrazole-4-yl)-(4-méthoxyphényle)cétone, la (3-amino-1H-pyrazole-4-yl)[3-(trifluorométhyl) phényl]cétone, la (3-amino-1H-pyrazole-4-yl)-(4-pyridyl)cétone, la (3-amino-1H-pyrazole-4-yl)-(2-thiényl)-cétone, la (3-amino-1H-5-méthyl-1H-pyrazole-4-yl)-phénylcétone, la (3-amino-1H-pyrazole-4-yl)-(4-méthylphényl)cétone, la (3-amino-1H-pyrazole-4-yl)-(2-pyridyl)cétone, la (3-amino-1H-pyrazole-4-yl)-(3-fluorophényl)cétone, la (3-amino-1H-pyrazole-4-yl)-(3-furyl)cétone, la (3-amino-1H-pyrazole-4-yl)-(3,4,5-triméthoxyphényl)cétone, la (3-amino-1H-pyrazole-4-yl)-(3,4-diméthoxyphényl)cétone, la (3-amino-1H-pyrazole-4-yl)-(3-méthylphényl)cétone, la (3-amino-1H-pyrazole-4-yl)-(3,5-diméthoxyphényl)cétone ou la (3-amino-1H-pyrazole-4-yl)-(2-fluorophényl)cétone.

3. Un procédé de la fabrication d'un composé de formule :

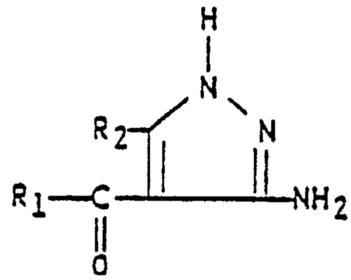

dans laquelle $R_1$ et $R_2$ sont définis à la revendication 1, qui consiste à faire réagir un acétonitrile substitué de manière convenable de formule :

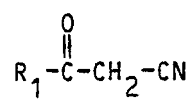

dans laquelle $R_1$ est comme décrit ci-dessus avec un diméthylacétal de N,N-diméthylamide produisant, après une réaction exothermique, un solide cristallisé, à récupérer ledit solide cristallisé, à le dissoudre dans le chlorure de méthylène, à faire passer sur du silicate de magnésium hydraté, à ajouter de l'hexane à l'éluat au reflux, à précipiter un [(α-diméthylamino)méthylène]-β-oxoarylpropionitrilede formule

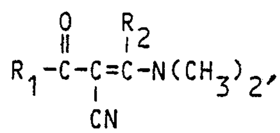

à faire réagir avec un sel d'aminoguanidine dans une solution d'hydroxyde de sodium 10N dans un alcanol inférieur au reflux pendant 6-10 h, ce qui donne le produit désiré.

Revendication pour l'Etat contractant suivant: AT

**1.** Un procédé de fabrication d'un composé de formule :

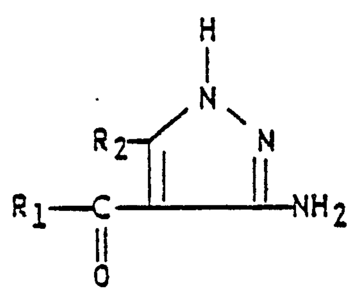

dans laquelle $R_1$ est choisi parmi un groupe phényle substitué par 1 ou 2 substituants choisis parmi les halogènes et les groupes alkyles en $C_1$-$C_3$ et les groupes alcoxy en $C_1$-$C_3$ ; un groupe phényle substitué par un substituant choisi parmi un grope dialkylamino en $C_1$-$C_3$, un groupe méthylènedioxy, un groupe alkylthio en $C_1$-$C_3$, un Groupe alkylsulfonyle en $C_1$-$C_3$, un groupe arylsulfonyle substitué, un groupe amino, un groupe alcanoylamino en $C_1$-$C_3$, un groupe aroylamino substitué, un groupe trifluorométhyle et un groupe phényle ; un groupe pyridyle ; un groupe pyridyle substitué par 1 ou 2 substituants choisis parmi les halogènes et les groupes alkyles en $C_1$-$C_3$ et alcoxy en $C_1$-$C_3$ ; le groupe thiényle ; un groupe thiényle substitué par 1 ou 2 substituants choisis parmi les halogènes et les groupes alkyles en $C_1$-$C_3$ et alcoxy en $C_1$-$C_3$ ; un groupe furyle ; un groupe naphtyle ; et un groupe pyrazinyle ; et $R_2$ est choisi parmi l'hydrogène et les groupes alkyles en $C_1$-$C_3$, qui consiste à faire réagir un acétonitrile substitué de manière convenable de formule :

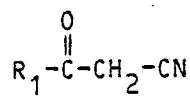

dans laquelle $R_1$ est comme décrit ci-dessus avec un diméthylacétal de N,N-diméthylamide produisant, après une réaction exothermique, un solide cristallisé, à récupérer ledit solide cristallisé, à le dissoudre dans le chlorure de méthylène, à faire passer sur du silicate de magnésium hydraté, à ajouter de l'hexane à l'éluat au reflux, à précipiter un [(α-diméthylamino)méthylène]-β-oxoarylpropionitrile de formule

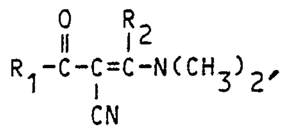

à faire réagir avec un sel d'aminoguanidine dans une solution d'hydroxyde de sodium 10N dans un alcanol inférieur au reflux pendant 6-10 h, ce qui donne le produit désiré.

## Ansprüche

1. Verbindung der Formel

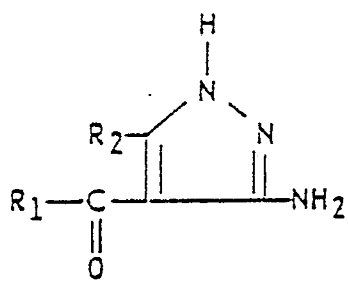

wobei $R_1$ für Phenyl, substituiert mit einem oder zwei von Halogen, Alkyl-($C_1$-$C_3$) oder Alkoxy-($C_1$-$C_3$); Phenyl , substituiert mit einem von Dialkylamino-($C_1$-$C_3$), Methylendioxy, Alkylthio-($C_1$-$C_3$), Alkylsulfonyl-($C_1$-$C_3$), substituiertes Arylsulfonyl, Amino, Alkanoyl-($C_1$-$C_3$)-amino, substituiertes Aroylamino, Trifluormethyl oder Phenyl; Pyridinyl; Pyridinyl, substituiert mit einem oder zwei von Halogen, Alkyl-($C_1$-$C_3$) oder Alkoxy($C_1$-$C_3$); Thienyl; Thienyl, substituiert mit einem oder zwei von Halogen, Alkyl-($C_1$ -$C_3$) oder Alkoxy-($C_1$ -$C_3$); Furanyl; Naphthalenyl; oder Pyrazinyl steht; und $R_2$ für Wasserstoff oder Alkyl-($C_1$ -$C_3$) steht.

2. Verbindung gemäß Anspruch 1, nämlich
(3-Amino-1H-pyrazol-4-yl)-(2-furanyl)-methanon;
(3-Amino-1H-pyrazol-4-yl)-(4-chlorphenyl)-methanon;
(3-Amino-1H-pyrazol-4-yl)-(4-fluorphenyl)-methanon;
(3-Amino-1H-pyrazol-4-yl)-(4-methoxyphenyl)-methanon;
(3-Amino-1H-pyrazol-4-yl)-[3-(trifluormethyl)-phenyl]-methanon;
(3-Amino-1H-pyrazol-4-yl)-(4-pyridinyl-methanon;
(3-Amino-1H-pyrazol-4-yl)-(2-thienyl)-methanon;
(3-Amino-5-methyl-1H-pyrazol-4-yl)-phenylmethanon;
(3-Amino-1H-pyrazol-4-yl)-(4-methylphenyl)-methanon;
(3-Amino-1H-pyrazol-4-yl)-(2-pyridinyl)-methanon;
(3-Amino-1H-pyrazol-4-yl)-(3-fluorphenyl)-methanon;
(3-Amino-1H-pyrazol-4-yl)-(3-furanyl)-methanon;
(3-Amino-1H-pyrazol-4-yl)-(3,4,5-trimethoxyphenyl)-methanon;

(3-Amino-1H-pyrazol-4-yl)-(3,4-dimethoxyphenyl)-methanon;
(3-Amino-1H-pyrazol-4-yl)-(3-methylphenyl)-methanon;
(3-Amino-1H-pyrazol-4-yl)-(3,5-dimethoxyphenyl)-methanon; oder
(3-Amino-1H-pyrazol-4-yl)-(2-fluorphenyl)-methanon.

3. Verfahren zur Herstellung einer Verbindung der Formel

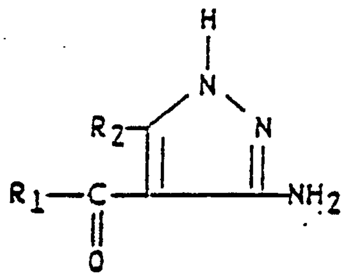

wobei $R_1$ und $R_2$ wie in Anspruch 1 definiert sind, umfassend die Umsetzung eines zweckentsprechend substituierten Acetonitrils der Formel

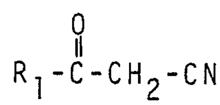

(wobei $R_1$ die oben angegebene Bedeutung hat) mit einem N,N-Dimethylamid-dimethylacetal, um nach einer exothermen Reaktion einen kristallinen Feststoff zu erzeugen, Isolierung des kristallinen Feststoffs, Auflösung in Methylenchlorid, Durchleiten durch wässriges Magnesiumsilikat, Zugabe von Hexan zu dem refluxierenden Eluat, Präzipitation eines [(α -Dimethylamino)-methylen]-β-oxoarylpropannitrils der Formel

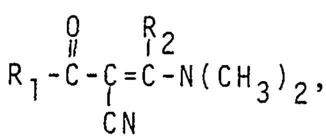

Umsetzung mit Aminoguanidinsalz in einer Niederalkanollösung von 10N Natriumhydroxid bei Rückfluß während 6 bis 10 Stunden, um das gewünschte Produkt zu erhalten.

Patentanspruch für folgenden Vertragsstaat: AT

1. Verfahren zur Herstellung einer Verbindung der Formel

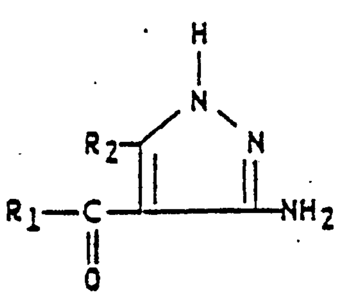

wobei $R_1$ für Phenyl, substituiert mit einem oder zwei von Halogen, Alkyl-($C_1$-$C_3$) oder Alkoxy-($C_1$-$C_3$); Phenyl, substituiert mit einem von Dialkylamino-($C_1$-$C_3$), Methylendioxy, Alkylthio-($C_1$-$C_3$), Alkylsulfonyl-($C_1$-$C_3$), substituiertes Arylsulfonyl, Amino, Alkanoyl-($C_1$-$C_3$)-amino, substituiertes Aroylamino, Trifluormethyl oder Phenyl; Pyridinyl; Pyridinyl, substituiert mit einem oder zwei von Halogen, Alkyl-($C_1$-$C_3$)

oder Alkoxy($C_1$-$C_3$); Thienyl; Thienyl, substituiert mit einem oder zwei von Halogen, Alkyl-($C_1$-$C_3$) oder Alkoxy-($C_1$-$C_3$); Furanyl; Naphthalenyl; oder Pyrazinyl steht; und $R_2$ für Wasserstoff oder Alkyl-($C_1$-$C_3$) steht, umfassend die Umsetzung eines zweckentsprechend substituierten Acetonitrils der Formel

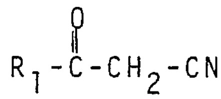

(wobei $R_1$ die oben angegebene Bedeutung hat) mit einem N,N-Dimethylamid-dimethylacetal, um nach einer exothermen Reaktion einen kristallinen Feststoff zu erzeugen, Isolierung des kristallinen Feststoffs, Auflösung in Methylenchlorid, Durchleiten durch wässriges Magnesiumsilikat, Zugabe von Hexan zu dem refluxierenden Eluat, Präzipitation eines [(α-Dimethylamino)-methylen]-β-oxoarylpropannitrils der Formel

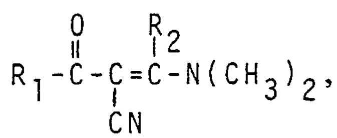

Umsetzung mit Aminoguanidinsalz in einer Niederalkanollösung von 10N Natriumhydroxid bei Rückfluß während 6 bis 10 Stunden, um das gewünschte Produkt zu erhalten.